# EUROPEAN PATENT SPECIFICATION

(11) **EP 2 393 880 B1**
(45) Date of publication and mention of the grant of the patent: **07.01.2015**
(21) Application number: 10739197.1
(22) Date of filing: 05.02.2010
(51) Int. Cl.: C08L 89/00, C09J 201/06, A61L 24/04, C09J 171/02, C08G 81/00, C08L 77/06, C09J 177/06

(54) **MULTI-LINKED STAR-SHAPED POLYMERS AND SYNTHETIC METHODS THEREFOR**
MEHRFACH VERNETZTE STERNFÖRMIGE POLYMERE UND SYNTHETISCHE VERFAHREN DAFÜR
POLYMÈRES EN FORME D'ÉTOILE À LIAISONS MULTIPLES ET LEURS PROCÉDÉS DE SYNTHÈSE

(30) Priority: 06.02.2009 US 150464 P
(43) Date of publication of application: 14.12.2011
(73) Proprietor: Kensey Nash Corporation, Exton, Pennsylvania 19341 (US)
(72) Inventor: LEE, Bruce P., Madison Wisconsin 53719 (US); SILVARY, Sunil, Quadribagh, Amberpet Hyderabad, AP (IN); MURPHY, John L., Verona WI 53593 (US)
(74) Representative: Glawe, Delfs, Moll
(86) International application number: PCT/US2010/023385
(87) International publication number: WO 2010/091302

(56) References cited:
- WO-A1-2008/019352
- WO-A1-2010/037045
- WO-A1-2010/091298
- US-A1- 2003 087 338
- US-A1- 2005 288 398
- US-A1- 2006 241 281

## Description

### CROSS REFERENCE TO RELATED APPLICATIONS

### FIELD OF THE INVENTION

The invention relates generally to polymer blends with multihydroxy (dihydroxy) phenyl derivatives (DHPDs).

### BACKGROUND OF THE INVENTION

Mussel adhesive proteins (MAPs) are remarkable underwater adhesive materials secreted by certain marine organisms which form tenacious bonds to the substrates upon which they reside. During the process of attachment to a substrate, MAPs are secreted as adhesive fluid precursors that undergo a crosslinking or hardening reaction which leads to the formation of a solid adhesive plaque. One of the unique features of MAPs is the presence of L-3-4-dihydroxyphenylalanine (DOPA), an unusual amino acid which is believed to be responsible for adhesion to substrates through several mechanisms that are not yet fully understood. The observation that mussels adhere to a variety of surfaces in nature (metal, metal oxide, polymer) led to a hypothesis that DOPA-containing peptides can be employed as the key components of synthetic medical adhesives or coatings.Polymers with dihydroxyphenyl moieties are disclosed in US 2005/0288398 A1.

For example, bacterial attachment and biofilm formation are serious problems associated with the use of urinary stents and catheters as they often lead to chronic infections that cannot be resolved without removing the device. Although numerous strategies have been employed to prevent these events including the alteration of device surface properties, the application of anti-attachment and antibacterial coatings, host dietary and urinary modification, and the use of therapeutic antibiotics, no one approach has yet proved completely effective. This is largely due to three important factors, namely various bacterial attachment and antimicrobial resistance strategies, surface masking by host urinary and bacterial constituents, and biofilm formation. While the urinary tract has multiple anti-infective strategies for dealing with invading microorganisms, the presence of a foreign stent or catheter provides a novel, non-host surface to which they can attach and form a biofilm. This is supported by studies highlighting the ability of normally non-uropathogenic microorganisms to readily cause device-associated urinary tract infections. Ultimately, for a device to be clinically successful it must not only resist bacterial attachment but that of urinary constituents as well. Such a device would better allow the host immune system to respond to invading organisms and eradicate them from the urinary tract.

For example, bacterial attachment and subsequent infection and encrustation of uropathogenic *E. coli* (UPEC) cystitis is a serious condition associated with biofouling. Infections with *E. coli* comprise over half of all urinary tract device-associated infections, making it the most prevalent pathogen in such episodes.

Additionally, in the medical arena, few adhesives exist which provide both robust adhesion in a wet environment and suitable mechanical properties to be used as a tissue adhesive or sealant. For example, fibrin-based tissue sealants (e.g. Tisseel VH, Baxter Healthcare) provide a good mechanical match for natural tissue, but possess poor tissue-adhesion characteristics. Conversely, cyanoacrylate adhesives (e.g. Dermabond, ETHICON, Inc.) produce strong adhesive bonds with surfaces, but tend to be stiff and brittle in regard to mechanical properties and tend to release formaldehyde as they degrade.

Therefore, a need exists for materials that overcome one or more of the current disadvantages.

### BRIEF SUMMARY OF THE INVENTION

The present invention surprisingly provides multi-armed, multihydroxy (dihydroxy) phenyl derivatives (DHPDs) having the general formula (I): 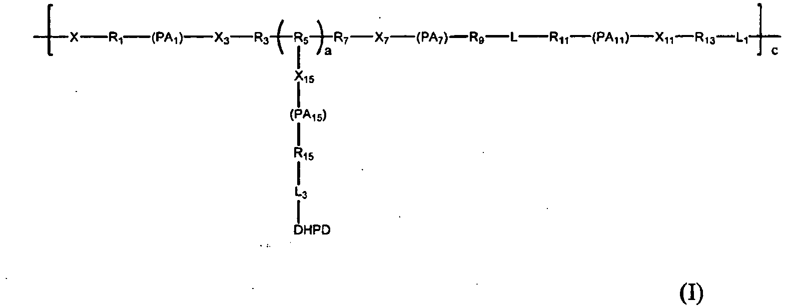 wherein:
each X, X₃, X₇, X₁₁ and X₁₅, independently, is O or NR;
each R, if present, is H or a branched or unbranched C1-C15 alkyl group;
each R₁, R₃, R₅, R₇, R₉, R₁₁, R₁₃ and R₁₅, independently, is a branched or unbranched C1-C15 alkyl group;
each PA₁, PA₇, PA₁₁ and PA₁₅, independently, is a residue of a substantially poly(alkylene oxide) polyether or derivative thereof;
each L, L₁ and L₃, independently, is a linking group selected from amine, amide, ether, ester, urea, carbonate or urethane linkages;
each DHPD, independently, is one of 3,4-dihydroxyphenethylamine (dopamine), 3,4-dihydroxyhydrocinnamic acid, 3,4-dihydroxyphenylethanol, 3, 4 dihydroxyphenylacetic acid, 3,4 dihydroxyphenylamine, or 3,4-dihydroxybenzoic acid
a is a value from 4 to 8; and
c is a value from 3 to 80.

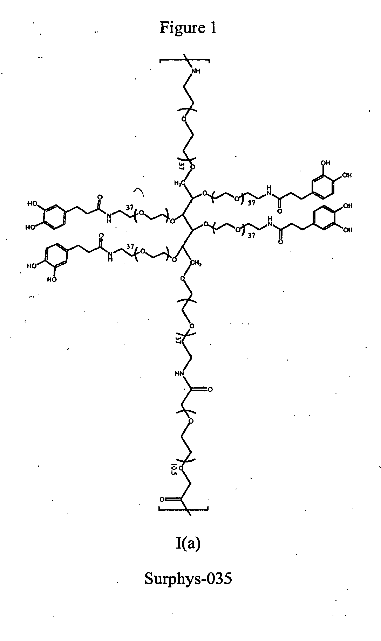

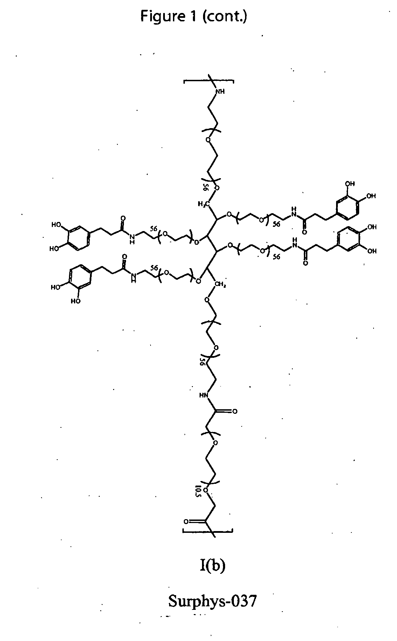

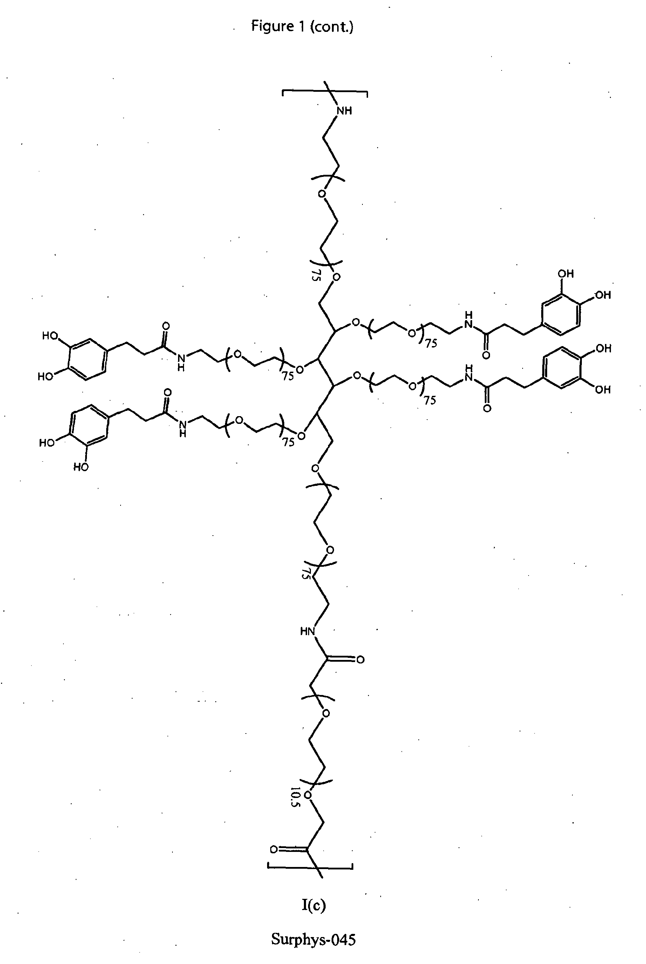

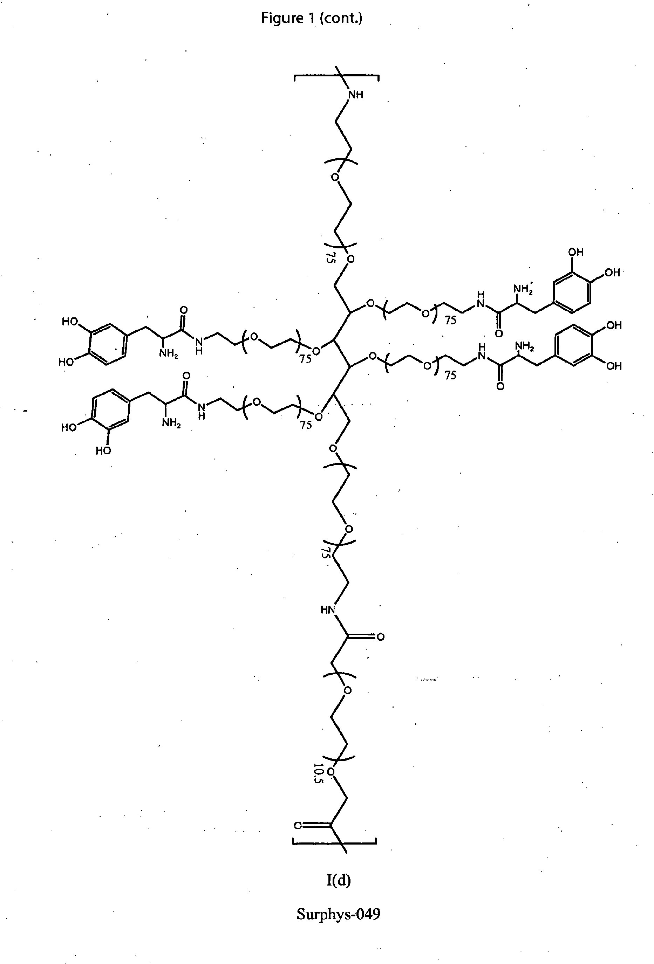

In one aspect, X is NH, X₃, X₇, X₁₁ and X₁₅, are each O, R₁, R₉, and R₁₅ are each -CH₂CH₂-, PA₁, PA₇, and PA₁₅ are each a residue of a substantially poly(alkylene oxide) polyether or derivative thereof wherein the repeat unit of the poly(ethylene oxide) polyether is about 37, about 56, or about 75 units, PA₁₁ is a residue of a substantially poly(alkylene oxide) polyether or derivative thereof wherein the repeat unit of the poly(ethylene oxide) polyether is about 10, R₅ is CH, R₃, R₇, R₁₁ and R₁₃ are each -CH₂-, and L, L₁ and L₃ are amide linkages, each DHPD is DOHA and "a" is 4 or 6. See for example Figure 1, compounds I(a) through I(d).

The compounds of the invention can be applied to a suitable substrate surface as a film or coating. Application of the compound(s) to the surface inhibits or reduces the growth of biofilm (bacteria) on the surface relative to an untreated substrate surface. In other embodiments, the compounds of the invention can be employed as an adhesive.

In one embodiment, adhesive compounds of the present invention provide a method of adhering a first surface to a second surface in a subject. In some embodiments, the first and second surfaces are tissue surfaces, for example, a natural tissue, a transplant tissue, or an engineered tissue. In further embodiments, at least one of the first and second surfaces is an artificial surface. In some embodiments, the artificial surface is an artificial tissue. In other embodiments, the artificial surface is a device or an instrument. In some embodiments, adhesive compounds of the present invention seal a defect between a first and second surface in a subject. In other embodiments, adhesive compounds of the present invention provide a barrier to, for example, microbial contamination, infection, chemical or drug exposure, inflammation, or metastasis. In further embodiments, adhesive compounds of the present invention stabilize the physical orientation of a first surface with respect to a second surface. In still further embodiments, adhesive compounds of the present invention reinforce the integrity of a first and second surface achieved by, for example, sutures, staples, mechanical fixators, or mesh. In some embodiments, adhesive compounds of the present invention provide control of bleeding. In other embodiments, adhesive compounds of the present invention provide delivery of drugs including, for example, drugs to control bleeding, treat infection or malignancy, or promote tissue regeneration.

Exemplary applications include, but are not limited to fixation of synthetic (resorbable and non-resorbable) and biological membranes and meshes for hernia repair , void-eliminating adhesive for reduction of post-surgical seroma formation in general and cosmetic surgeries, fixation of synthetic (resorbable and non-resorbable) and biological membranes and meshes for tendon and ligament repair, sealing incisions after ophthalmic surgery, sealing of venous catheter access sites, bacterial barrier for percutaneous devices, as a contraceptive device, a bacterial barrier and/or drug depot for oral surgeries (e.g. tooth extraction, tonsillectomy, cleft palate, etc.), for articular cartilage repair, for antifouling or anti-bacterial adhesion.

In one embodiment, the reaction products of the syntheses described herein are included as compounds or compositions useful as adhesives or surface treatment/antifouling aids. It should be understood that the reaction product(s) of the synthetic reactions can be purified by methods known in the art, such as diafiltration, chromatography, recrystallization/precipitation and the like or can be used without further purification.

It should be understood that the compounds of the invention can be coated multiple times to form bi, tri, etc. layers. The layers can be of the compounds of the invention per se, or of blends of a compound(s) and polymer, or combinations of a compound layer and a blend layer, etc.

Consequently, constructs can also include such layering of the compounds per se, blends thereof, and/or combinations of layers of a compound(s) per se and a blend or blends.

While multiple embodiments are disclosed, still other embodiments of the present invention will become apparent to those skilled in the art from the following detailed description. As will be apparent, the invention is capable of modifications in various obvious aspects, all without departing from the spirit and scope of the present invention. Accordingly, the detailed descriptions are to be regarded as illustrative in nature and not restrictive.

### BRIEF DESCRIPTION OF THE DRAWINGS

Figure 1 provides several formulaic embodiments of the invention.
Figure 2 shows percent reduction in adhered bacteria on a polyurethane material used in the manufacture of dental unit water lines coated with Surphys polymers. Culture conditions are in TSB Media
Figure 3 shows percent reduction in adhered bacteria on Surphys-coated TiO₂ surfaces. Culture conditions are in TSB Media
Figure 4 shows percent reduction in adhered bacteria on Surphys-coated Cook urinary stent polyurethane using human urine pool as culture media. Culture medium is human urine, pooled (HUP).
Figure 5 shows percent reduction in adhered bacteria on Surphys-coated TiO₂ surfaces in TSB media.
Figure 6 shows percent reduction in adhered bacteria on Surphys-coated PU surfaces in PSB media.
Figure 7 shows percent reduction in adhered bacteria on Surphys-coated polyurethane or PDMS using human urine pool as culture media. Culture medium is human urine, pooled (HUP).
Figure 8 shows percent reduction in adhered bacteria on Surphys-coated polyurethane or PDMS using human urine pool as culture media. Culture medium is human urine, pooled (HUP).

### DETAILED DESCRIPTION

In the specification and in the claims, the terms "including" and "comprising" are open-ended terms and should be interpreted to mean "including, but not limited to.... " These terms encompass the more restrictive terms "consisting essentially of" and "consisting of."

It must be noted that as used herein and in the appended claims, the singular forms "a", "an", and "the" include plural reference unless the context clearly dictates otherwise. As well, the terms "a" (or "an"), "one or more" and "at least one" can be used interchangeably herein. It is also to be noted that the terms "comprising", "including", "characterized by" and "having" can be used interchangeably.

Unless defined otherwise, all technical and scientific terms used herein have the same meanings as commonly understood by one of ordinary skill in the art to which this invention belongs. All publications and patents specifically mentioned herein are incorporated by reference in their entirety for all purposes including describing and disclosing the chemicals, instruments, statistical analyses and methodologies which are reported in the publications which might be used in connection with the invention. All references cited in this specification are to be taken as indicative of the level of skill in the art. Nothing herein is to be construed as an admission that the invention is not entitled to antedate such disclosure by virtue of prior invention.

"Alkyl," by itself or as part of another substituent, refers to a saturated or unsaturated, branched, straight-chain or cyclic monovalent hydrocarbon radical derived by the removal of one hydrogen atom from a single carbon atom of a parent alkane, alkene or alkyne. Typical alkyl groups include, but are not limited to, methyl; ethyls such as ethanyl, ethenyl, ethynyl; propyls such as propan-1-yl, propan-2-yl, cyclopropan-1-yl, prop-1-en-1-yl, prop-1-en-2-yl, prop-2-en-1-yl (allyl), cycloprop-1-en-1-yl; cycloprop-2-en-1-yl, prop-1-yn-1-yl , prop-2-yn-1-yl, *etc.;* butyls such as butan-1-yl, butan-2-yl, 2-methyl-propan-1-yl, 2-methyl-propan-2-yl, cyclobutan-1-yl, but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, but-1-yn-1-yl, but-1-yn-3-yl, but-3-yn-1-yl, *etc.;* and the like.

The term "alkyl" is specifically intended to include groups having any degree or level of saturation, *i.e.,* groups having exclusively single carbon-carbon bonds, groups having one or more double carbon-carbon bonds, groups having one or more triple carbon-carbon bonds and groups having mixtures of single, double and triple carbon-carbon bonds. Where a specific level of saturation is intended, the expressions "alkanyl," "alkenyl," and "alkynyl" are used. Preferably, an alkyl group comprises from 1 to 15 carbon atoms (C₁-C₁₅ alkyl), more preferably from 1 to 10 carbon atoms (C₁-C₁₀ alkyl) and even more preferably from 1 to 6 carbon atoms (C₁-C₁₆ alkyl or lower alkyl).

"Alkanyl," by itself or as part of another substituent, refers to a saturated branched, straight-chain or cyclic alkyl radical derived by the removal of one hydrogen atom from a single carbon atom of a parent alkane. Typical alkanyl groups include, but are not limited to, methanyl; ethanyl; propanyls such as propan-1-yl, propan-2-yl (isopropyl), cyclopropan-1-yl, etc.; butanyls such as butan-1-yl, butan-2-yl (sec-butyl), 2-methyl-propan-1-yl (isobutyl), 2-methyl-propan-2-yl (t-butyl), cyclobutan-1-yl, *etc*.; and the like.

"Alkenyl," by itself or as part of another substituent, refers to an unsaturated branched, straight-chain or cyclic alkyl radical having at least one carbon-carbon double bond derived by the removal of one hydrogen atom from a single carbon atom of a parent alkene. The group may be in either the *cis* or *trans* conformation about the double bond(s). Typical alkenyl groups include, but are not limited to, ethenyl; propenyls such as prop-1-en-1-yl, prop-1-en-2-yl, prop-2-en-1-yl (allyl), prop-2-en-2-yl, cycloprop-1-en-1-yl; cycloprop-2-en-1-yl; butenyls such as but-1-en-1-yl, but-1-en-2-yl, 2-methyl-prop-1-en-1-yl, but-2-en-1-yl, but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, *etc*.; and the like.

"Alkyldiyl" by itself or as part of another substituent refers to a saturated or unsaturated, branched, straight-chain or cyclic divalent hydrocarbon group derived by the removal of one hydrogen atom from each of two different carbon atoms of a parent alkane, alkene or alkyne, or by the removal of two hydrogen atoms from a single carbon atom of a parent alkane, alkene or alkyne. The two monovalent radical centers or each valency of the divalent radical center can form bonds with the same or different atoms. Typical alkyldiyl groups include, but are not limited to, methandiyl; ethyldiyls such as ethan-1,1-diyl, ethan-1,2-diyl, ethen-1,1-diyl, ethen-1,2-diyl; propyldiyls such as propan-1,1-diyl, propan-1,2-diyl, propan-2,2-diyl, propan-1,3-diyl, cyclopropan-1,1-diyl, cyclopropan-1,2-diyl, prop-1-en-1,1-diyl, prop-1-en-1,2-diyl, prop-2-en-1,2-diyl, prop-1-en-1,3-diyl, cycloprop-1-en-1,2-diyl, cycloprop-2-en-1,2-diyl, cycloprop-2-en-1,1-diyl, prop-1-yn-1,3-diyl, etc.; butyldiyls such as, butan-1,1-diyl, butan-1,2-diyl, butan-1,3-diyl, butan-1,4-diyl, butan-2,2-diyl, 2-methyl-propan-1,1 -diyl, 2-methyl-propan-1,2-diyl, cyclobutan-1,1-diyl; cyclobutan-1,2-diyl, cyclobutan-1,3-diyl, but-1-en-1,1-diyl, but-1-en-1,2-diyl, but-1-en-1,3-diyl, but-1-en-1,4-diyl, 2-methyl-prop-1-en-1,1-diyl, 2-methanylidene-propan-1,1-diyl, buta-1,3-dien-1,1-diyl, buta-1,3-dien-1,2-diyl, buta-1,3-dien-1,3-diyl, buta-1,3-dien-1,4-diyl, cyclobut-1 -en-1,2-diyl, cyclobut-1-en-1,3-diyl, cyclobut-2-en-1,2-diyl, cyclobuta-1,3-dien-1,2-diyl, cyclobuta-1,3-dien-1,3-diyl, but-1-yn-1,3-diyl, but-1-yn-1,4-diyl, buta-1,3-diyn-1,4-diyl, etc.; and the like. Where specific levels of saturation are intended, the nomenclature alkanyldiyl, alkenyldiyl and/or alkynyldiyl is used. Where it is specifically intended that the two valencies are on the same carbon atom, the nomenclature "alkylidene" is used. In preferred embodiments, the alkyldiyl group comprises from 1 to 6 carbon atoms (C1-C6 alkyldiyl). Also preferred are saturated acyclic alkanyldiyl groups in which the radical centers are at the terminal carbons, *e.g*., methandiyl (methano); ethan-1,2-diyl (ethano); propan-1,3-diyl (propano); butan-1,4-diyl (butano); and the like (also referred to as alkylenos, defined *infra*).

"Alkyleno," by itself or as part of another substituent, refers to a straight-chain saturated or unsaturated alkyldiyl group having two terminal monovalent radical centers derived by the removal of one hydrogen atom from each of the two terminal carbon atoms of straight-chain parent alkane, alkene or alkyne. The locant of a double bond or triple bond, if present, in a particular alkyleno is indicated in square brackets. Typical alkyleno groups include, but are not limited to, methano; ethylenos such as ethano, etheno, ethyno; propylenos such as propano, prop[1]eno, propa[1,2]dieno, prop[1]yno, etc.; butylenos such as butano, but[1]eno, but[2]eno, buta[1,3]dieno, but[1]yno, but[2]yno, buta[1,3]diyno, etc.; and the like. Where specific levels of saturation are intended, the nomenclature alkano, alkeno and/or alkyno is used. In preferred embodiments, the alkyleno group is (C1-C6) or (C1-C3) alkyleno. Also preferred are straight-chain saturated alkano groups, *e.g.,* methano, ethano, propano, butano, and the like.

"Alkylene" by itself or as part of another substituent refers to a straight-chain saturated or unsaturated alkyldiyl group having two terminal monovalent radical centers derived by the removal of one hydrogen atom from each of the two terminal carbon atoms of straight-chain parent alkane, alkene or alkyne. The locant of a double bond or triple bond, if present, in a particular alkylene is indicated in square brackets. Typical alkylene groups include, but are not limited to, methylene (methano); ethylenes such as ethano, etheno, ethyno; propylenes such as propano, prop[1]eno, propa[1,2]dieno, prop[1]yno, etc.; butylenes such as butano, but[1]eno, but[2]eno, buta[1,3]dieno, but[1]yno, but[2]yno, buta[1,3]diyno, etc.; and the like. Where specific levels of saturation are intended, the nomenclature alkano, alkeno and/or alkyno is used. In preferred embodiments, the alkylene group is (C1-C6) or (C1-C3) alkylene. Also preferred are straight-chain saturated alkano groups, *e.g.,* methano, ethano, propano, butano, and the like.

"Substituted," when used to modify a specified group or radical, means that one or more hydrogen atoms of the specified group or radical are each, independently of one another, replaced with the same or different substituent(s). Substituent groups useful for substituting saturated carbon atoms in the specified group or radical include, but are not limited to -R^{a}, halo, -O⁻, =O, -OR^{b}, -SR^{b}, -S⁻, =S, -NR^{c}R^{c}, =NR^{b}, =N-OR^{b}, trihalomethyl, -CF₃, -CN, -OCN, -SCN, -NO, -NO₂, =N₂, -N₃, -S(O)₂R^{b}, -S(O)₂O⁻, -S(O)₂OR^{b}, -OS(O)₂R^{b}, -OS(O)₂O⁻, -OS(O)₂OR^{b}, -P(O)(O⁻)₂, -P(O)(OR^{b})(O⁻), -P(O)(OR^{b})(OR^{b}), -C(O)R^{b}, -C(S)R^{b}, -C(NR^{b})R^{b}, -C(O)O⁻, -C(O)OR^{b}, -C(S)OR^{b}, -C(O)NR^{c}R^{c}, -C(NR^{b})NR^{c}R^{c}, -OC(O)R^{b}, -OC(S)R^{b}, -OC(O)O⁻, -OC(O)OR^{b}, -OC(S)OR^{b}, -NR^{b}C(O)R^{b}, -NR^{b}C(S)R^{b}, -NR^{b}C(O)O⁻, -NR^{b}C(O)OR^{b}, -NR^{b}C(S)OR^{b}, -NR^{b}C(O)NR^{c}R^{c}, -NR^{b}C(NR^{b})R^{b} and -NR^{b}C(NR^{b})NR^{c}R^{c}, where R^{a} is selected from the group consisting of alkyl, cycloalkyl, heteroalkyl, cycloheteroalkyl, aryl, arylalkyl, heteroaryl and heteroarylalkyl; each R^{b} is independently hydrogen or R^{a}; and each R^{c} is independently R^{b} or alternatively, the two R^{c}s are taken together with the nitrogen atom to which they are bonded form a 5-, 6- or 7-membered cycloheteroalkyl which may optionally include from 1 to 4 of the same or different additional heteroatoms selected from the group consisting of O, N and S. As specific examples, -NR^{c}R^{c} is meant to include NH₂, -NH-alkyl, N-pyrrolidinyl and N-morpholinyl.

Similarly, substituent groups useful for substituting unsaturated carbon atoms in the specified group or radical include, but are not limited to, -R^{a}, halo, -O⁻, -OR^{b}, -SR^{b}, -S⁻, -NR^{c}R^{c}, trihalomethyl, -CF₃, -CN, -OCN,-SCN, -NO, -NO₂, -N₃, -S(O)₂R^{b}, -S(O)₂O⁻, -S(O)₂OR^{b}, -OS(O)₂R^{b}, -OS(O)₂O⁻, -OS(O)₂OR^{b}, -P(O)(O⁻)₂, -P(O)(OR^{b})(O⁻), -P(O)(OR^{b})(OR^{b}), -C(O)R^{b}, -C(S)R^{b}, -C(NR^{b})R^{b}, -C(O)O⁻, -C(O)OR^{b}, -C(S)OR^{b}, -C(O)NR^{c}R^{c}, -C(NR^{b})NR^{c}R^{c}, -OC(O)R^{b}, -OC(S)R^{b}, -OC(O)O⁻, -OC(O)OR^{b}, -OC(S)OR^{b}, -NR^{b}C(O)R^{b}, -NR^{b}C(S)R^{b}, -NR^{b}C(O)O⁻, -NR^{b}C(O)OR^{b}, -NR^{b}C(S)OR^{b}, -NR^{b}C(O)NR^{c}R^{c}, -NR^{b}C(NR^{b})R^{b} and -NR^{b}C(NR^{b})NR^{c}R^{c}, where R^{a}, R^{b} and R^{c} are as previously defined.

Substituent groups useful for substituting nitrogen atoms in heteroalkyl and cycloheteroalkyl groups include, but are not limited to, -R^{a}, -O⁻, -OR^{b}, -SR^{b}, -S⁻, -NR^{c}R^{c}, trihalomethyl, -CF₃, -CN, -NO, -NO₂, -S(O)₂R^{b}, -S(O)₂O⁻, -S(O)₂OR^{b}, -OS(O)₂R^{b}, -OS(O)₂O⁻, -OS(O)₂OR^{b}, -P(O)(O⁻)₂, -P(O)(OR^{b})(O⁻), -P(O)(OR^{b})(OR^{b}), -C(O)R^{b}, -C(S)R^{b}, -C(NR^{b})R^{b}, -C(O)OR^{b}, -C(S)OR^{b}, -C(O)NR^{c}R^{c}, -C(NR^{b})NR^{c}R^{c}, -OC(O)R^{b}, -OC(S)R^{b}, -OC(O)OR^{b}, -OC(S)OR^{b}, -NR^{b}C(O)R^{b}, -NR^{b}C(S)R^{b}, -NR^{b}C(O)OR^{b}, -NR^{b}C(S)OR^{b}, -NR^{b}C(O)NR^{c}R^{c}, -NR^{b}C(NR^{b})R^{b} and -NR^{b}C(NR^{b})NR^{c}R^{c}, where R^{a}, R^{b} and R^{c} are as previously defined.

Substituent groups from the above lists useful for substituting other specified groups or atoms will be apparent to those of skill in the art.

The substituents used to substitute a specified group can be further substituted, typically with one or more of the same or different groups selected from the various groups specified above.

The identifier "PA" refers to a poly(alkylene oxide) or substantially poly(alkylene oxide) and means predominantly or mostly alkyloxide or alkyl ether in composition. This definition contemplates the presence of heteroatoms e.g., N, O, S, P, etc. and of functional groups e.g., -COOH, -NH₂, - SH, or -OH as well as ethylenic or vinylic unsaturation. It is to be understood any such non-alkyleneoxide structures will only be present in such relative abundance as not to materially reduce, for example, the overall surfactant, non-toxicity, or immune response characteristics, as appropriate, of this polymer. It should also be understood that PAs can include terminal end groups such as PAO-CH₂-CH₂-NH₂, e.g., PEG-O-CH₂-CH₂-NH₂ (as a common form of amine terminated PA). PA-O-CH₂-CH₂-CH₂-NH₂, e.g., PEG-O-CH₂-CH₂-CH₂-NH₂ is also available as well as PA-O-(CH₂-CH(CH₃)-O)ₓₓ-CH₂-CH(CH₃)-NH₂, where xx is 0 to about 3, e.g., PEG-O-(CH₂-CH(CH₃)-O)ₓₓ-CH₂-CH(CH₃)-NH₂ and a PA with an acid end-group typically has a structure of PA-O-CH₂-COOH, e.g., PEG-O-CH₂-COOH or PA-O-CH₂-CH₂-COOH, e.g., PEG-O-CH₂-CH₂-COOH. These can be considered "derivatives" of the PA. These are all contemplated as being within the scope of the invention and should not be considered limiting.

Suitable PAs (polyalkylene oxides) include polyethylene oxides (PEOs), polypropylene oxides (PPOs), polyethylene glycols (PEGs) and combinations thereof that are commercially available from SunBio Corporation, JenKem Technology USA, NOF America Corporation or Creative PEGWorks. It should be understood that, for example, polyethylene oxide can be produced by ring opening polymerization of ethylene oxide as is known in the art.

In one embodiment, the PA can be a block copolymer of a PEO and PPO or a PEG or a triblock copolymer of PEO/PPO/PEO.

Suitable MW ranges of the PA's are from about 300 to about 8,000 daltons, 400 to about 5,000 daltons or from about 450 to about 3,500 daltons.

It should be understood that the PA terminal end groups can be functionalized. Typically the end groups are OH, NH₂, COOH, or SH. However, these groups can be converted into a halide (Cl, Br, I), an activated leaving group, such as a tosylate or mesylate, an ester, an acyl halide, N-succinimidyl carbonate, 4-nitrophenyl carbonate, and chloroformate with the leaving group being N-hydroxy succinimide, 4-nitrophenol, and Cl, respectively, etc.

The notation of "L" refers to a linking group which is the reaction product of the terminal end moieties, for example, of a PA and DHPD condense to form an amide, ether, ester, urea, carbonate or urethane linkage depending on the reactive sites on the PA and DHPD. In other words, a bond is formed between the PA and DHPD portion of the molecule.

The term "residue" is used to mean that a portion of a first molecule reacts (e.g., condenses or is an addition product via a displacement reaction) with a portion of a second molecule to form, for example, a linking group, such an amide, ether, ester, urea, carbonate or urethane linkage depending on the reactive sites on the PA and DHPD. This can be referred to as "linkage". Examples would be the condensation product of a carboxylic acid and an alcohol, such as a terminal hydroxyl of a PA and the carboxylic acid of a DHPD. Alternatively, condensation of an amine with a carboxylic ester would form an amide bond. Similarly, this also includes forming ether linkages between two hydroxyl groups by, for example, substitution reactions, dehydration, or epoxide openings.

It should be understood that a person having ordinary skill in the art would select appropriate combinations of moieties to provide an array of condensation products embodied and described herein.

It should be understood, that upon condensation of the DHPD molecule with the PA that a molecule of water, for example, is generated such that a bond is formed as described above (amide, ether, ester, urea, carbonate or urethane).

DHPD molecules include 3, 4-dihydroxyphenethylamine (dopamine), 4-dihydroxyhydrocinnamic acid, 3,4-dihydroxyphenyl ethanol, 3,4 dihydroxyphenylacetic acid, 3, 4 dihydroxyphenyenylamine, 3,4-dihydroxybenzoic acid,.

The present invention surprisingly provides multi-armed, multihydroxy (dihydroxy) phenyl derivatives (DHPDs) having the general formula (I) 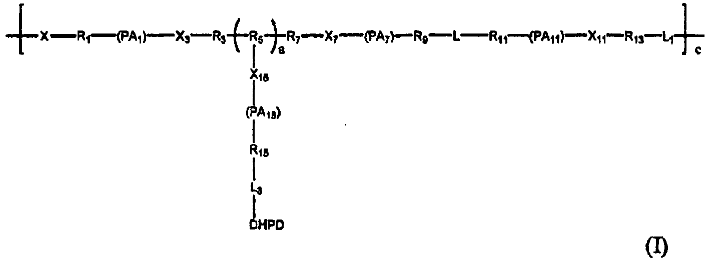 wherein:
each X, X₃, X₇, X₁₁ and X₁₅, independently, is O or NR;
each R, if present, is H or a branched or unbranched C1-C15 alkyl group;
each R₁, R₃, R₅, R₇, R₉, R₁₁, R₁₃ and R₁₅, independently, is a branched or unbranched C1-C15 alkyl group;
each PA₁, PA₇, PA₁₁ and PA₁₅, independently, is a residue of a substantially poly(alkylene oxide) polyether or derivative thereof;
each L, L₁ and L₃, independently, is a linking group selected from amine, amide, ether, ester, urea, carbonate or urethane linkages;
each DHPD, independently: is one of 3,4-dihydroxyphenethylamine (dopamine), 3,4-dihydroxyhydrocinnamic acid, 3,4-dihydroxyphenylethanol, 3,4 dihydroxyphenylacetic acid, 3,4 dihydroxyphenylamine, or 3,4-dihydroxybenzoic acid
a is a value from 4 to 8 (e.g., 4 etc through 8); and
c is a value from 3 to 80 (e.g., 3, etc. through 80).

In one embodiment X is NR and R is H.

In another embodiment each X₃, X₇, X₁₁ and X₁₅ is O.

In still another embodiment each R₁, R₉ and R₁₅ are -CH₂CH₂-,

In yet another embodiment, each PA₁, PA₇ and PA₁₅, independently, is a residue of a substantially poly(ethylene oxide) polyether and in particular the repeat unit of the poly(ethylene oxide) polyether is about 37, about 56, or about 75 units.

In another embodiment PA₁₁ is a residue of a substantially poly(ethylene oxide) polyether and in particular the poly(ethylene oxide) polyether is about 10 units.

In still another embodiment R₃ and R₇ are each -CH₂-.

In yet another embodiment each R₅ is a -CH.

In still yet another embodiment each L, L₁ and L₃ is an amide linkage.

In another embodiment each DHPD residue is one of 3,4-dihydroxyhydrocinnamic acid (DOHA), 3, 4-dihydroxyphenyl ethanol, 3, 4 dihydroxyphenylacetic acid, 3, 4 dihydroxyphenylamine, or 3,4-dihydroxybenzoic acid.

In an embodiment c is a value of 3 to 25.

The invention further provides crosslinked hydrogels derived from the compositions described herein. For example, two DHPD moieties from two separate polymer chains can be reacted to form a bond between the two DHPD moieties. Typically, this is an oxidative/radical initiated crosslinking reaction wherein oxidants/initiators such as periodates and iodates, such as NaIO₄ or KIO₄, NaIO₃ or KIO₃ and the like, FeCl₃, H₂O₂, oxygen, an inorganic base, an organic base or an enzymatic oxidase can be used. Typically, a ratio of oxidant/initiator to DHPD containing material is between about 0.2 to about 1.0 (on a molar basis) (oxidant:DHPD). In one particular embodiment, the ratio is between about 0.25 to about 0.75 and more particularly between about 0.4 to about 0.6 (e.g., 0.5). It has been found that periodate is very effective in the preparation of crosslinked hydrogels of the invention.

In still another aspect, blends of the compounds of the invention described herein, can be prepared with various polymers. Polymers suitable for blending with the compounds of the invention are selected to impart non-covalent interactions with the compound(s), such as hydrophobic-hydrophobic interactions or hydrogen bonding with an oxygen atom on PEG and a substrate surface. These interactions can increase the cohesive properties of the film to a substrate. If a biopolymer is used, it can introduce specific bioactivity to the film, (i.e. biocompatibility, cell binding, immunogenicity, etc.).

Suitable polymers include, for example, polyesters, PPG, linear PCL-diols (MW 600-2000), branched PCL-triols (MW 900), wherein PCL can be replaced with PLA, PGA, PLGA, and other polyesters, amphiphilic block (di, tri, or multiblock) copolymers of PEG and polyester or PPG, tri-block copolymers of PCL-PEG-PCL (PCL MW = 500 - 3000, PEG MW = 500 - 3000), tri-block copolymers of PLA-PEG-PLA (PCL MW = 500 - 3000, PEG MW = 500 - 3000), wherein PCL and PLA can be replaced with PGA, PLGA, and other polyesters. Pluronic polymers (triblock, diblock of various MW) and other PEG, PPG block copolymers are also suitable. Hydrophilic polymers with multiple functional groups (-OH, -NH2, -COOH) contained within the polymeric backbone such as PVA (MW 10,000-100,000), poly acrylates and poly methacrylates, polyvinylpyrrolidone, and polyethylene imines are also suitable. Biopolymers such as polysaccharides (e.g., dextran), hyaluronic acid, chitosan, gelatin, cellulose (e.g., carboxymethyl cellulose), proteins, etc. which contain functional groups can also be utilized.

Abbreviations: PCL = polycaprolactone, PLA= polylactic acid, PGA= Polyglycolic acid, PLGA= a random copolymer of lactic and glycolic acid, PPG=polypropyl glycol, and PVA= polyvinyl alcohol.

Typically, blends of the invention include from about 0 to about 99.9% percent (by weight) of polymer to composition(s) of the invention, more particularly from about 1 to about 50 and even more particularly from about 1 to about 30.

The compositions of the invention, either a blend or a compound of the invention per se, can be applied to suitable substrates using conventional techniques. Coating, dipping, spraying, spreading and solvent casting are possible approaches.

The present invention surprisingly provides unique antifouling coatings/constructs that are suitable for application in, for example, urinary applications. The coatings could be used anywhere that a reduction in bacterial attachment is desired: dental unit waterlines, implantable orthopedic devices, cardiovascular devices, wound dressings, percutaneous devices, surgical instruments, marine applications, food preparation surfaces and utensils.

The present invention surprisingly provides unique bioadhesive constructs that are suitable to repair or reinforce damaged tissue.

Suitable supports include those that can be formed from natural materials, such as collagen, metal surfaces such as titanium, iron, steel, etc. or man made materials such as polypropylene, polyethylene, polybutylene, polyesters, PTFE, PVC, polyurethanes and the like. The support can be a solid surface such as a film, sheet, coupon or tube, a membrane, a mesh, a non-woven and the like. The support need only help provide a surface for the coating to adhere.

Other suitable supports can be formed from a natural material, such as collagen, pericardium, dermal tissues, small intestinal submucosa and the like. The support can be a film, a membrane, a mesh, a non-woven and the like. The support need only help provide a surface for the bioadhesive/coating to adhere. The support should also help facilitate physiological reformation of the tissue at the damaged site. Thus the constructs of the invention provide a site for remodeling via fibroblast migration, followed by subsequent native collagen deposition. For biodegradable support of either biological or synthetic origins, degradation of the support and the adhesive can result in the replacement of the bioadhesive construct by the natural tissues of the patient.

The coatings of the invention can include a compound of the invention or mixtures thereof or a blend of a polymer with one or more of the compounds of the invention. In one embodiment, the construct is a combination of a substrate, to which a blend is applied, followed by a layer(s) of one or more compounds of the invention.

In another embodiment, two or more layers can be applied to a substrate wherein the layering can be combinations of one or more blends or one or more compositions of the invention. The layering can alternate between a blend and a composition layer or can be a series of blends followed by a composition layer or vice versa.

It has interestingly been found that use of a blend advantageously has improved adhesion to the substrate surface. For example, a blend of a hydrophobic polymer with a composition of the invention of Formula I should have improved adhesion to a hydrophobic substrate. Subsequent application of a composition of Formula I to the blend layer then provides improved interfacial adhesion between the blend and provides for improved adhesive properties to the tissue to be adhered to as the hydrophobic polymer is not in the outermost layer.

Typically the loading density of the coating layer is from about 0.001 g/m² to about 200 g/m², more particularly from about 5 g/m² to about 150 g/m², and more particularly from about 10 g/m² to about 100 g/m². Thus, typically a coating has a thickness of from about 1 to about 200 nm. More typically for an adhesive, the thickness of the film is from about 1 to about 200 microns.

The following paragraphs enumerated consecutively from 1 through 32 provide for various aspects of the present invention. In one embodiment, in a first paragraph (1), the present invention provides A compound comprising the formula (I) 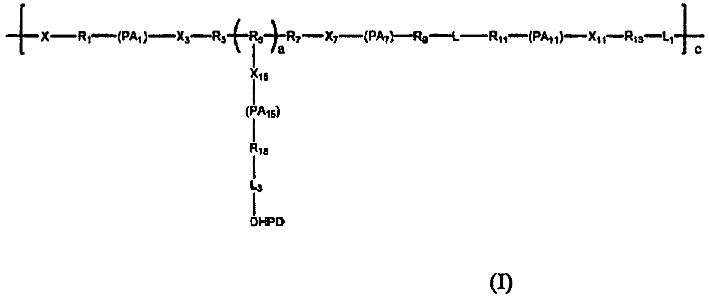 wherein:
each X, X₃, X₇, X₁₁ and X₁₅, independently, is O or NR;
each R, if present, is H or a branched or unbranched C1-C15 alkyl group;
each R₁, R₃, R₅, R₇, R₉, R₁₁, R₁₃ and R₁₅, independently, is a branched or unbranched C1-C15 alkyl group;
each PA₁, PA₇, PA₁₁ and PA₁₅, independently, is a residue of a substantially poly(alkylene oxide) polyether or derivative thereof;
each L, L₁ and L₃, independently, is a linking group selected from amine, amide, ether, ester, urea, carbonate or urethane linkages;
each DHPD, independently: is one of 3,4-dihydroxyphenethylamine (dopamine), 3,4-dihydroxyhydrocinnamic acid, 3, 4-dihydroxyphenylethanol, 3, 4 dihydroxyphenylacetic acid, 3, 4 dihydroxyphenylamine, or 3, 4-dihydroxybenzoic acid
a is a value from 4 to 8; and
c is a value from 3 to 80.

2. The compound of paragraph 1, wherein X is NR and R is H.
3. The compound of either of paragraphs 1 or 2, wherein each X₃, X₇, X₁₁ and X₁₅ is O.
4. The compound of any of paragraphs 1 through 3, wherein each R₁, R₉ and R₁₅ are -CH₂CH₂-.
5. The compound of any of paragraphs 1 through 4, wherein each PA₁, PA₇ and PA₁₅, independently, is a residue of a substantially poly(ethylene oxide) polyether.
6. The compound of paragraph 5, wherein the repeat unit of the poly(ethylene oxide) polyether is about 37, about 56, or about 75 units.
7. The compound of any of paragraphs 1 through 6, wherein PA₁₁ is a residue of a substantially poly(ethylene oxide) polyether.
8. The compound of paragraph 7, wherein the repeat unit of the poly(ethylene oxide) polyether is about 10 units.
9. The compound of any of paragraphs 1 through 8, wherein R₃, R₇, R₁₁ and R₁₃ are each -CH₂-.
10. The compound of any of paragraphs 1 through 9, wherein each R₅ is a -CH.
11. The compound of any of paragraphs 1 through 10, wherein each L, L₁ and L₃ is an amide linkage.
12. The compound of any of paragraphs 1 through 11, wherein each DHPD residue is 3, 4-dihydroxyhydrocinnamic acid, (DOHA)
14. The compound of any of paragraphs 1 through 13, wherein a is a value of 4 to 8.
15. The compound of any of paragraphs 1 through 14, wherein c is a value of 3 to 25.
16. A bioadhesive construct, comprising:
   a support suitable for tissue repair or reconstruction; and
   a coating comprising a multihydroxyphenyl (DHPD) functionalized polymer (DHPp) of any of paragraphs 1 through 15.
17. The bioadhesive construct of paragraph 26, further comprising an oxidant.
18. The bioadhesive construct of either of paragraphs 16 or 17, wherein the oxidant is formulated with the coating.
19. The bioadhesive construct of either of paragraphs 16 or 17, wherein the oxidant is applied to the coating.
20. The bioadhesive construct of any of paragraphs 16 through 19, wherein the support is a film, a mesh, a membrane, a nonwoven or a prosthetic.
21. A blend of a polymer and a compound of any of paragraphs 1 through 15.
22. The blend of paragraph 21, wherein the polymer is present in a range of about 1 to about 50 percent by weight.
23. The blend of paragraph 22, wherein the polymer is present in a range of about 1 to about 30 percent by weight.
24. A bioahesive construct comprising:
   a support suitable for tissue repair or reconstruction; and
   a coating comprising any of the blends of paragraphs 21 through 23.
25. The bioadhesive construct of paragraph 24, further comprising an oxidant.
26. The bioadhesive construct of either of paragraphs 24 or 25, wherein the oxidant is formulated with the coating.
27. The bioadhesive construct of either of paragraphs 24 or 25, wherein the oxidant is applied to the coating.
28. The bioadhesive construct of any of paragraphs 24 through 27, wherein the support is a film, a mesh, a membrane, a nonwoven or a prosthetic.
29. A bioadhesive construct comprising:
   a support suitable for tissue repair or reconstruction;
   a first coating comprising a multihydroxyphenyl (DHPD) functionalized polymer (DHPp) of any of paragraphs 1 through 15 and a polymer; and
   a second coating coated onto the first coating, wherein the second coating comprises a multihydroxyphenyl (DHPD) functionalized polymer (DHPp) of any of paragraphs 1 through 15.
30. A bioadhesive construct comprising:
   a support suitable for tissue repair or reconstruction;
   a first coating comprising a first multihydroxyphenyl (DHPD) functionalized polymer (DHPp) of any of paragraphs 1 through 15 and a first polymer; and
   a second coating coated onto the first coating, wherein the second coating comprises a second multihydroxyphenyl (DHPD) functionalized polymer (DHPp) of any of paragraphs 1 through 15 and a second polymer, wherein the first and second polymer may be the same or different and wherein the first and second DHPp can be the same or different.
31. A bioadhesive construct comprising:
   a support suitable for tissue repair or reconstruction;
   a first coating comprising a first multihydroxyphenyl (DHPD) functionalized polymer (DHPp) of any of paragraphs 1 through 15; and
   a second coating coated onto the first coating, wherein the second coating comprises a second multihydroxyphenyl (DHPD) functionalized polymer (DHPp) of any of paragraphs 1 through 15, wherein the first and second DHPp can be the same or different.
32. A method to reduce bacterial growth on a substrate surface, comprising the step of coating a multihydroxyphenyl (DHPD) functionalized polymer (DHPp) of any of paragraphs 1 through 15 onto the surface of the substrate.

The invention will be further described with reference to the following non-limiting Examples. It will be apparent to those skilled in the art that many changes can be made in the embodiments described without departing from the scope of the present invention. Thus the scope of the present invention should not be limited to the embodiments described in this application, but only by embodiments described by the language of the claims and the equivalents of those embodiments. Unless otherwise indicated, all percentages are by weight.

### Examples

### Materials and Method Development

### 1.1. Syntheses

### Example 1: Synthesis of Surphys-035

Dissolved 10 g of 6-arm PEG-NH₂ (10,000 MW; 1 mmol), 600 mg of PEG-bCME (Mn ~600, 1 mmol), and 911 mg of DOHA (5 mmol) with 40 ml chloroform and 20 ml DMF in a round bottom flask equipped with an addition funnel. Added 946 mg of HOBt (7 mmol), 2.65 g of HBTU (7 mmol), and 840 µL of triethylamine (6 mmol) in 30 mL of DMF dropwise to the round bottom flask over a period of 90 minutes. Stirred at room temperature for 2 hours. Added the mixture to 600 mL of diethyl ether. The precipitate was collected via vacuum filtration and dried. The crude product was further purified through dialysis (15,000 MWCO) in deionized H₂O (acidified to pH 3.5) for 24 hrs. After lyophilization, 6.1 g of Surphys-035 was obtained. ¹H NMR (400 MHz, D₂O): δ 6.84-6.66 (m, 3H, C₆*H₃*(OH)₂-), 4.09 (s, 2H, PEG-*CH₂*-O-C(O)-NH-), 3.87-3.29 (m, PEG), 2.8 (t, 2H, C₆H₃(OH)₂-*CH₂*-CH₂-C(O)-NH-), 2.48 (t, 2H, C₆H₃(OH)₂-CH₂-*CH₂*-C(O)-NH-),.UV-vis spectroscopy: 0.29 ± 0.0040 µmole DH/mg polymer (DOHA) (4.8 ± 0.00074 wt% DH). GPC : Mw = 61,000, Mn = 28,000, PD = 2.2

### Example 2: Synthesis of Surphys-037

Dissolved 15 g of 6-arm PEG-NH₂ (15,000 MW; 1 mmol), 600 mg of PEG-bCME (Mn ~600, 1 mmol), and 911 mg of DOHA (5 mmol) with 40 ml chloroform and 20 ml DMF in a round bottom flask equipped with an addition funnel. Added 946 mg of HOBt (7 mmol), 2.65 g of HBTU (7 mmol), and 840 µL of triethylamine (6 mmol) in 30 mL of DMF dropwise to the round bottom flask over a period of 90 minutes. Stirred at room temperature for 2 hours. Added the mixture to 600 mL of diethyl ether. The precipitate was collected via vacuum filtration and dried. The crude product was further purified through dialysis (15,000 MWCO) in deionized H₂O (acidified to pH 3.5) for 24 hrs. After lyophilization, 12 g of Surphys-037 was obtained. ¹H NMR (400 MHz, D₂O): δ 6.71-6.54 (m, 3H, C₆*H₃*(OH)₂-), 4.72 (s, 2H, PEG-*CH₂*-O-C(O)-NH-), 3.96-3.15 (m, PEG), 2.67 (t, 2H, C₆H₃(OH)₂-*CH₂*-CH₂-C(O)-NH-), 2.37 (t, 2H, C₆H₃(OH)₂-CH₂-*CH₂*-C(O)-NH-),.UV-vis spectroscopy: 0.202 ± 0.0029 µmole DH/mg polymer (3.34 ± 0.05 wt% DH). GPC : Mw = 316,200, Mn = 68,690 , PD = 4.6.

### Example 3: Synthesis of Surphys-045

Dissolved 10 g of 6-arm PEG-NH₂ (20,000 MW; 0.5 mmol) 300 mg of poly(ethyleneglycol) bis (carboxymethyl) ether average Mn ~600 ( 0.5 mmol), and 455 mg of 3,4-dihydroxyhydrocinnamic acid (2.5 mmol) in 40 ml chloroform and 20 ml DMF in a round bottom flask equipped with an addition funnel. Added 473 mg of HOBt (3.5 mmol), 1.32 g of HBTU (3.5 mmol), 30 mL DMF and 416 µL of triethylamine (3 mmol) to the addition funnel and this mixture was added dropwise to the round bottom flask over a period of 90 minutes. After stirring at room temperature for 2 hrs, the mixture was added to 900 mL of diethyl ether. The precipitate was collected via filtration and dried with vacuum pump. The crude product was further purified using dialysis (15,000 MWCO) in deionized H₂O (acidified to pH 3.5) for 24 hrs. The polymer was obtained through lyophilization. ¹H NMR (400 MHz, D₂O): δ 6.82-6.74 (m, 3H, C₆*H₃*(OH)₂-), 4.0 (s, 2H, PEG-*CH₂*-O-C(O)-NH-), 3.88-3.51 (m, PEG), 2.80 (t , 2H, C₆H₃(OH)₂-*CH₂*-CH₂-C(O)-NH-), 2.50 (t, 2H, C₆H₃(OH)₂-CH₂-*CH₂*-C(O)-NH-),.UV-vis spectroscopy: 0.141 ± 0.0026 µmole DH/mg polymer (2.33 ± 0.04 wt% DH). GPC : Mw = 164,000, Mn = 58,770, PD = 2.7.

### Example 4: Synthesis of Surphys-049

Added 10 g of 6-arm PEG-NH₂ (20,000 MW; 0.5 mmol), to 300 mg of poly(ethyleneglycol) bis(carboxymethyl)ether average Mn ~600 ( 0.5 mmol), 328 mg of N-Boc-Dopa (2.5 mmol), 40 ml chloroform and 20 ml DMF in a round bottom flask equipped with an addition funnel. Stirred the mixture at room temperature to dissolve. Added 473 mg of HOBt (3.5 mmol), 1.32 g of HBTU (3.5 mmol), 30 mL DMF and 416 µL of triethylamine (3 mmol) to the addition funnel and added this mixture dropwise to the round bottom flask over a period of 90 minutes. Continued stirring for additional 2 hours. Added the mixture to 900 mL of diethyl ether, then collected the precipitate via filtration and dried. Dissolved crude product in 40 ml chloroform and 40 ml of trifluoroacetic acid and stirred for half hour at room temperature, and solvent was evaporated in vacuo. The remaining crude product was dissolved in 70 ml of chloroform and poured into 800 ml of diethyl ether. The precipitate was collected via vacuum filtration and dried. The crude product is then purified further through dialysis (15,000 MWCO) in deionized H₂O (acidified to pH 3.5) for 48 hrs. After lyophilization, both ¹H NMR and UV-vis was used to determine the coupling efficiency of the catechol to PEG.

¹H NMR (400 MHz, D₂O): δ 6.79-6.59 (m, 3H, C₆*H₃*(OH)₂-), 3.9 (s, 2H, PEG-*CH₂*-O-C(O)-NH-), 3.76-3.41 (m, PEG), 3.11 (t, 2H, C₆H₃(OH)₂-CH₂-C*H*(NH₂)-C(O)-NH-), 2.96-2.89 (m, 2H, C₆H₃(OH)₂-*CH₂*-CH(NH₂)-C(O)-NH-),.UV-vis spectroscopy: 0.184 ± 0.014 µmole DM/mg polymer (3.32 ± 0.26 wt% DM). GPC : Mw = 77,420, Mn = 42,500 , PD = 1.8.

### Molecular Weight Determination using Gel Permeation Chromatography (GPC)

Molecular weight of polymers described herein were determined by gel permeation chromatography in concert with triple-angle laser light scattering on a Optilab® rEX (Wyatt Technology) refractive index detector and a miniDAWN^{™} TREOS (Wyatt Technology) triple-angle light scattering detector using Shodex-OH Pak columns (SB-804 HQ and SB-802.5 HQ) in a mobile phase of 50:50 mixture of methanol and phosphate buffered saline. For the molecular weight calculation, the experimentally determined reflective index (dn/dc) value of the polymer was used.

### Coating Method

Coatings used: Surphys-002 (S002), Surphys-035 (S035), Surphys-037 (S037), and Surphys-045 (S045)

### Coating Conditions:

S002: 0.6M K₂SO₄, 0.1 M MOPS, pH 9 at 50°C MOPS = morpholinopropanesulfonic acid

S035 and S037: 0.3M K₂SO₄, 0.05M MOPS, pH 6, 50°C

S045: 0.4M K₂SO₄, 0.0667M MOPS, pH 6, 40°C

Titanium oxide test materials were cleaned by sequential 10 minute sonication in 5% phosphate-free detergent, ultrapure water, acetone and 2-propanol. Polyurethane substrates (e.g., water line tubes and urinary stents) were cleaned by sequential 10 minute sonication in 5% phosphate-free detergent, ultrapure water and 70% ethanol. Test materials were coated with antifouling polymer by immersion in a I mg/mL concentration of the polymer/buffer solution and incubated for 24 hours at the lower critical solution temperature (LCST) to maximize surface coverage. After coating, samples were rinsed twice with deionized water and dried in a stream of nitrogen gas.

To determine the antifouling ability of these coatings, bacterial cell attachment and biofilm formation was assessed on both coated and uncoated samples. *Staphylococcus aureus* cultures were grown overnight in tryptic soy broth (TSB) at 37°C to a concentration of 1x10⁹. Bacteria cultures were resuspended in TSB the following day and diluted to 1x10⁵. Triplicate samples of coated test materials were placed in 24 well plates and covered with a 1.5 mL suspension of *S. aureus* and incubated for 24 hours at 37°C. The samples were rinsed twice in PBS and the bacterial biofilm was removed using two sterile cotton swabs. The swabs were sonicated for 10 min in 5 mL PBS to remove adherent bacteria. The tubes containing the swabs and PBS solution were then vortexed for 1 min at 3000 rpm and dilution-plated onto tryptic soy agar (TSA) plates. The plates were incubated overnight at 37°C and quantified to attain absolute bacterial counts for all coated and control surfaces. All bacterial counts were standardized per mm² of exposed surface area of test substrates. Experiments were carried out in triplicate and assessed for significance.

### Results:

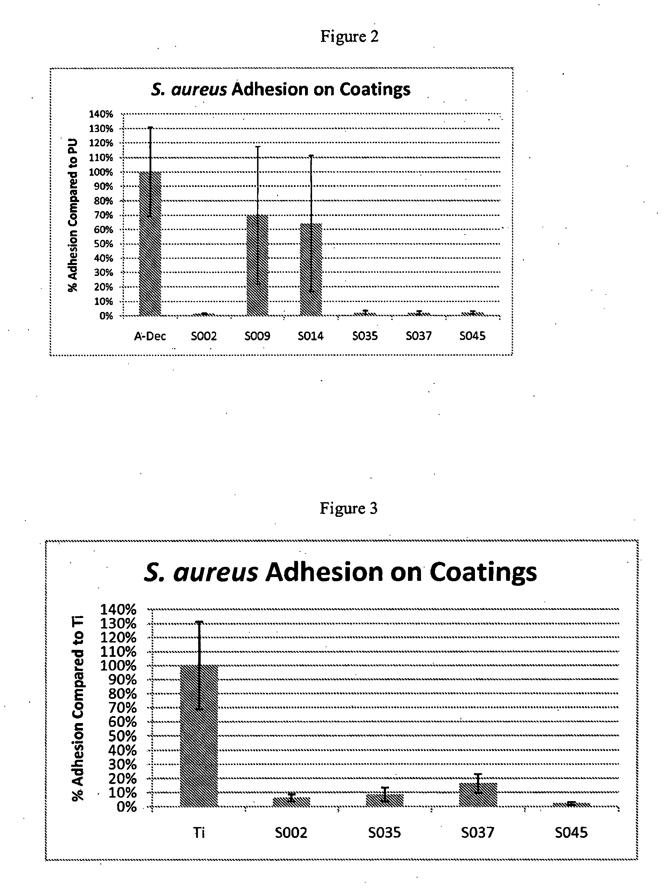

Figures 2 through 4 demonstrate the antifouling ability of the multibranched PEG-based polymers (S-035, S-037, S-045) on both Ti and polyurethane surfaces. In most cases, these multibranched coating reduced bacteria adhesion by at least 85% as compared to the uncoated surfaces. These coating also performed equally as well as S-002.

### 24-hr Bacteria testing protocol in TSB:

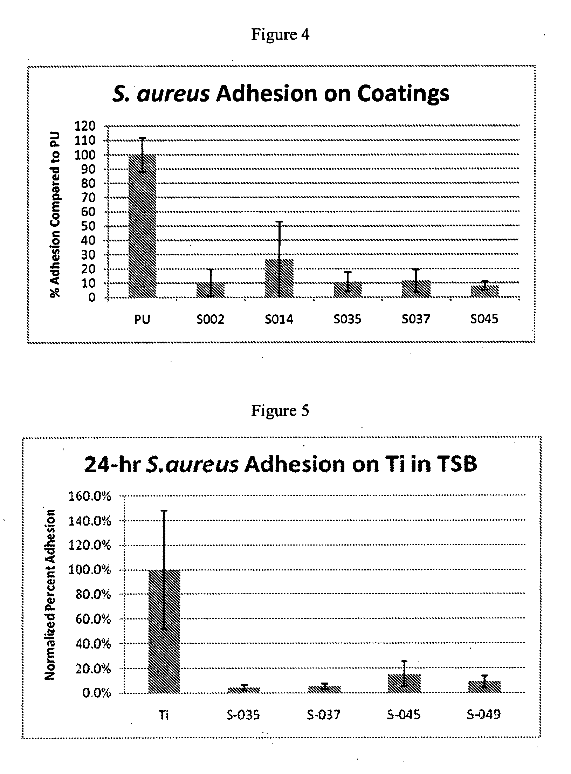

*Staphylococcus aureus* was grown overnight in batch culture at 37°C. After incubation, the bacteria were resuspended in TSB and diluted to ~1x10⁵ CFU/mL. Coated and uncoated Ti substrates were placed in 24-well plates were covered with 1.5 mL of bacterial suspension. The plates were incubated at 37 °C for 24 h. After incubation, the samples were rinsed twice with PBS and the biofilm was scraped off using two sterile cotton swabs subsequently placed in 15-mL centrifuge tubes containing 5 mL sterile PBS. The tubes were sonicated for 10 min to remove any bacteria adherent to the cotton swab and vortexed for 1 min to ensure a uniform suspension. Serial dilutions were made and 100 µL of each dilution plated onto tryptic soy agar (TSA) to determine the number of CFUs on each substrate. The results were normalized to the surface area of the test substrates and compared to uncoated control surfaces. As shown in Figure 5, Surphys-coated Ti demonstrated over 85% bacteria adhesion to control Ti surface.

### 24-hr Bacteria testing protocol in PBS:

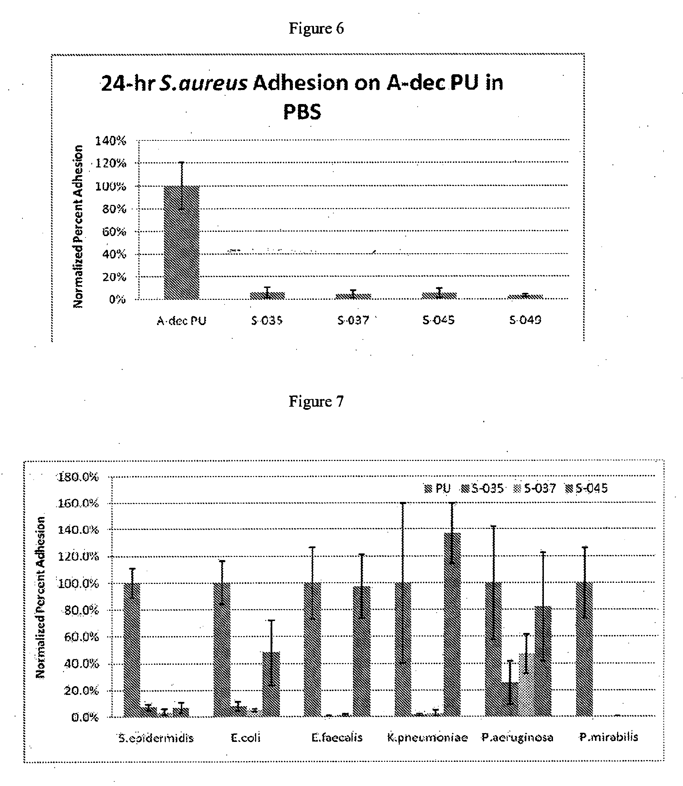

*Staphylococcus aureus* was grown overnight in batch culture at 37 C. After incubation, the bacteria were resuspended in 1xPBS and diluted to ~1 x 10⁷ CFU/mL. Coated and uncoated dental unit water line materials were placed in 24-well plates were covered with 2 mL of bacterial suspension. The plates were incubated at 20 °C for 24 h. After incubation, the samples were rinsed twice with PBS and the biofilm was scraped off using two sterile cotton swabs subsequently placed in 15-mL centrifuge tubes containing 5 mL sterile PBS. The tubes were sonicated for 10 min to remove any bacteria adherent to the cotton swab and vortexed for 1 min to ensure a uniform suspension. Serial dilutions were made and 100 µL of each dilution plated onto tryptic soy agar (TSA) to determine the number of CFUs on each substrate. The results were normalized to the surface area of the test substrates and compared to uncoated control surfaces. As shown in Figure 6, Surphys-coated polyurethane (PU) demonstrated over 93% bacteria adhesion to control PU surface.

### Bacteria testing protocol in pooled human urine

Six common uropathogens (Escherichia coli, Klebsiella pneumonia, Proteus mirabilis, Enterococcus faecalis, Staphylococcus epidermidis, and Pseudomonas aeruginosa) were obtained from ATCC (Manassas, VA). Urine was anonymously collected from Nerites employees after informed consent, using an IRB-approved protocol. Collected urine was pooled and adjusted to pH 6.5, followed by filter sterilization using 0.2-µm filters (Nalgene, Rochester, NY). Each bacterial strain was grown overnight in batch culture at 37°C. After incubation, the bacteria were resuspended in sterile urine and diluted to ~1 × 10⁵ colony-forming units (CFUs)/mL, a typical clinically relevant concentration.

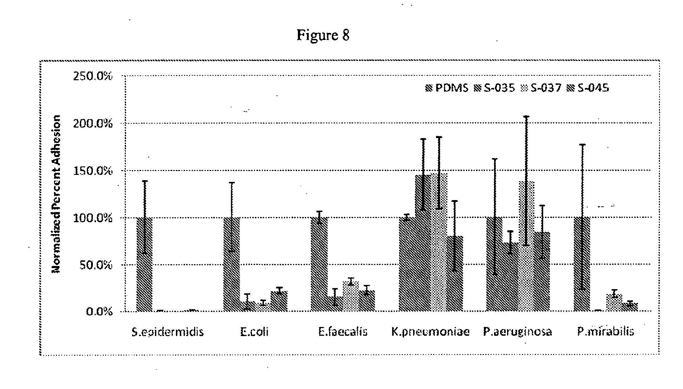

Coated and uncoated urinary stent and catheter materials (PU and PDMS, respectively) were placed in 24-well plates and covered with 1.5 mL of bacterial suspension. The plates were incubated at 37°C for 24 h. A 48-h incubation was used with P. mirabilis due to its low adherence to control substrates after 24 h. Following incubation, each sample was rinsed twice with 2-mL aliquots of sterile 1x phosphate-buffered saline (PBS) to remove any weakly adherent and non-adherent organisms. Adherent organisms were subsequently dislodged using two sterile cotton swabs that were then placed into 5 mL of sterile PBS, sonicated for 10 min, vortexed for 1 min, and dilution-plated onto tryptic soy agar or nutrient agar (Proteus mirabilis) plates. The plates were incubated overnight at 37°C and the number of CFUs on each substrate was quantified. The results were normalized to the surface area of the test substrates and compared to those for uncoated control surfaces. Bacterial adhesion results on Surphys-coated polyurethane (PU) and polydimethylsiloxane (PDMS) are shown in Figures 7 and 8, respectively.

## Claims

1. A compound comprising the formula (I) 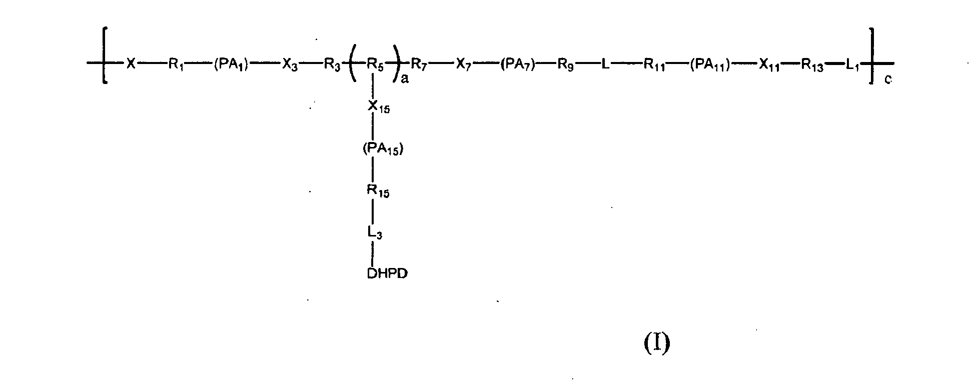 wherein:
each X, X₃, X₇, X₁₁ and X₁₅, independently, is O or NR;
each R, if present, is H or a branched or unbranched C1-C15 alkyl group;
each R₁, R₃, R₅, R₇, R₉, R₁₁, R₁₃ and R₁₅, independently, is a branched or unbranched C1-C15 alkyl group;
each PA₁, PA₇, PA₁₁ and PA₁₅, independently, is a residue of a substantially poly(alkylene oxide) polyether or derivative thereof;
each L, L₁ and L₃, independently, is a linking group selected from amine, amide, ether, ester, urea, carbonate or urethane linkages; each DHPD, independently, is one of 3, 4-dihydroxyphenethylamine (dopamine), 3, 4-dihydroxyhydrocinnamic acid, 3, 4-dihydroxyphenyl ethanol, 3, 4 dihydroxyphenylacetic acid, 3, 4 dihydroxyphenylamine, or 3, 4- dihydroxybenzoic acid:
a is a value from 4 to 8; and
c is a value from 3 to 80.

2. The compound of claim 1, wherein X is NR and R is H; and/or wherein each X₃, X₇, X₁₁ and X₁₅ is O; and/or wherein each R₁, R₉ and R₁₅ are -CH₂CH₂-.

3. The compound of claim 1 or 2, wherein each PA₁, PA₇ and PA₁₅, independently, is a residue of a substantially poly(ethylene oxide) polyether, wherein preferably the repeat unit of the poly( ethylene oxide) polyether is 37, 56, or 75 units.

4. The compound of any of claims 1 through 3, wherein PA₁₁ is a residue of a substantially poly(ethylene oxide) polyether, wherein preferably the repeat unit of the poly( ethylene oxide) polyether is 10 units.

5. The compound of any of claims 1 through 4, wherein R₃, R₇, R₁₁ and R₁₃ are each -CH₂-; and/or wherein each R₅ is a -CH; and/or wherein each L, L₁ and L₃ is an amide linkage.

6. The compound of any of claims 1 through 5, wherein each DHPD is a 3, 4- dihydroxyhydrocinnamic acid (DOHA) residue.

7. The compound of any of claims 1 through 6, wherein c is a value of 3 to 25.

8. A bioadhesive construct, comprising:
a support suitable for tissue repair or reconstruction;
and a coating comprising a multihydroxyphenyl (DHPD) functionalized polymer (DHPp) of any of claims 1 through 7, optionally further comprising an oxidant,
wherein preferably the oxidant is formulated with the coating or applied to the coating.

9. A blend of a polymer and a compound of any of claims 1 through 7, wherein preferably the polymer is present in a range of 1 to 50 percent by weight, further preferred 1 to 30 percent by weight.

10. A bioahesive construct comprising: a support suitable for tissue repair or reconstruction; and a coating comprising a blend of claim 9, optionally further comprising an oxidant, wherein preferably the oxidant is formulated with the coating or applied to the coating.

11. The bioadhesive construct of claim 8 or 10, wherein the support is a film, a mesh, a membrane, a nonwoven or a prosthetic.

12. A bioadhesive construct comprising: a support suitable for tissue repair or reconstruction; a first coating comprising a multihydroxyphenyl (DHPD) functionalized polymer (DHPp) of any of claims 1 through 7 and a polymer; and a second coating coated onto the first coating, wherein the second coating comprises a multihydroxyphenyl (DHPD) functionalized polymer (DHPp) of any of claims 1 through 7.

13. A bioadhesive construct comprising: a support suitable for tissue repair or reconstruction; a first coating comprising a first multihydroxyphenyl (DHPD) functionalized polymer (DHPp) of any of claims 1 through 7 and a first polymer; and a second coating coated onto the first coating, wherein the second coating comprises a second multihydroxyphenyl (DHPD) functionalized polymer (DHPp) of any of claims 1 through 7 and a second polymer, wherein the first and second polymer may be the same or different and wherein the first and second DHPp can be the same or different.

14. A bioadhesive construct comprising: a support suitable for tissue repair or reconstruction; a first coating comprising a first multihydroxyphenyl (DHPD) functionalized polymer (DHPp) of any of claims 1 through 7; and a second coating coated onto the first coating, wherein the second coating comprises a second multihydroxyphenyl (DHPD) functionalized polymer (DHPp) of any of claims 1 through 7, wherein the first and second DHPp can be the same or different.

15. A method to reduce bacterial growth on a substrate surface, comprising the step of coating a multihydroxyphenyl (DHPD) functionalized polymer (DHPp) of any of claims 1 through 7 onto the surface of the substrate.

## Patentansprüche

1. Verbindung umfassend die Formel (I), 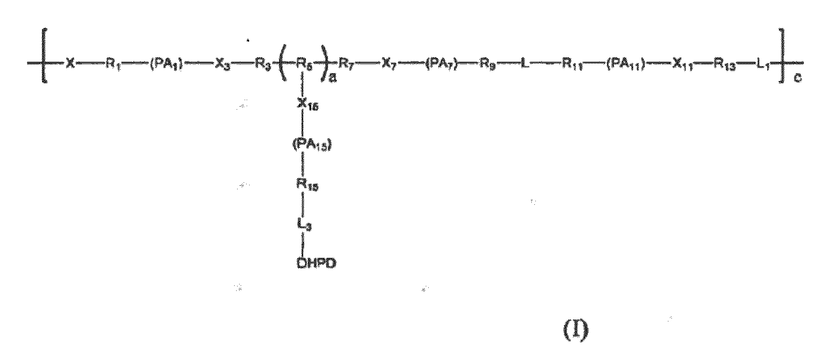 wobei:
jedes X, X₃, X₇, X₁₁ und X₁₅ unabhängig 0 oder NR ist;
jedes R, falls vorhanden, H oder eine verzweigte oder nichtverzweigte C1-C15-Alkylgruppe ist;
jedes R₁, R₃, R₅, R₇, R₉, R₁₁, R₁₃ und R₁₅ unabhängig eine verzweigte oder nichtverzweigte C1-C15-Alkylgruppe ist;
jedes PA₁, PA₇, PA₁₁ und PA₁₅ unabhängig ein Rest eines im Wesentlichen Poly(alkylenoxid)-Polyethers oder eines Derivats davon ist;
jedes L, L₁ und L₃ unabhängig eine Linkergruppe ausgewählt aus Amin-, Amid-, Ether-, Ester-, Harnstoff-, Carbonat- und Urethan-Verknüpfungen ist; jedes DHPD unabhängig eines von 3,4-Dihydroxyphenethylamin (Dopamin), 3,4-Dihydroxyhydrozimtsäure, 3,4-Dihydroxyphenylethanol, 3,4-Dihydroxyphenylessigsäure, 3,4-Dihydroxyphenylamin und 3,4-Dihydroxybenzoesäure ist;
a ein Wert von 4 bis 8 ist; und
c ein Wert von 3 bis 80 ist.

2. Verbindung gemäß Anspruch 1, wobei X NR ist und R H ist; und/oder wobei jedes X₃, X₇, X₁₁ und X₁₅ 0 ist; und/oder wobei jedes R₁, R₉ und R₁₅ -CH₂CH₂- ist.

3. Verbindung gemäß Anspruch 1 oder 2, wobei jedes PA₁, PA₇ und PA₁₅ unabhängig ein Rest eines im Wesentlichen Poly(ethylenoxid)-Polyethers ist, wobei vorzugsweise die Wiederholungseinheit des Poly(ethylenoxid)-Polyethers 37, 56 oder 75 Einheiten ist.

4. Verbindung gemäß einem der Ansprüche 1 bis 3, wobei PA₁₁ ein Rest eines im Wesentlichen Poly(ethylenoxid)-Polyethers ist, wobei vorzugsweise die Wiederholungseinheit des Poly(ethylenoxid)-Polyethers 10 Einheiten ist.

5. Verbindung gemäß einem der Ansprüche 1 bis 4, wobei R₃, R₇, R₁₁ und R₁₃ jeweils -CH₂- sind; und/oder wobei jedes R₅ ein -CH ist; und/oder wobei jedes L, L₁ und L₃ eine Amidverknüpfung ist.

6. Verbindung gemäß einem der Ansprüche 1 bis 5, wobei jedes DHPD ein 3,4-Dihydroxyhydrozimtsäure(DOHA)-Rest ist.

7. Verbindung gemäß einem der Ansprüche 1 bis 6, wobei c ein Wert von 3 bis 25 ist.

8. Bioklebstoffkonstrukt umfassend:
einen Träger, der für die Gewebereparatur oder -rekonstruktion geeignet ist;
und eine Beschichtung umfassend ein Multihydroxyphenyl(DHPD)-funktionalisiertes Polymer (DHPp) gemäß einem der Ansprüche 1 bis 7, gegebenenfalls ferner umfassend ein Oxidationsmittel, wobei das Oxidationsmittel vorzugsweise mit der Beschichtung formuliert oder auf die Beschichtung aufgebracht ist.

9. Gemisch eines Polymers und einer Verbindung gemäß einem der Ansprüche 1 bis 7, wobei vorzugsweise das Polymer im Bereich von 1 bis 50 Gewichtsprozent vorhanden ist, bevorzugter 1 bis 30 Gewichtsprozent.

10. Bioklebstoffkonstrukt umfassend: einen Träger, der für die Gewebereparatur oder -rekonstruktion geeignet ist; und eine Beschichtung umfassend ein Gemisch gemäß Anspruch 9, gegebenenfalls ferner umfassend ein Oxidationsmittel, wobei das Oxidationsmittel vorzugsweise mit der Beschichtung formuliert oder auf die Beschichtung aufgebracht ist.

11. Bioklebstoffkonstrukt gemäß Anspruch 8 oder 10, wobei der Träger ein Film, ein Netz, eine Membran, ein Vlies oder eine Prothese ist.

12. Bioklebstoffkonstrukt umfassend: einen Träger, der für die Gewebereparatur oder -rekonstruktion geeignet ist; eine erste Beschichtung umfassend ein Multihydroxyphenyl(DHPD)-funktionalisiertes Polymer (DHPp) gemäß einem der Ansprüche 1 bis 7 und ein Polymer; und eine zweite Beschichtung, die auf die erste Beschichtung geschichtet ist, wobei die zweite Beschichtung ein Multihydroxyphenyl(DHPD)-funktionalisiertes Polymer (DHPp) gemäß einem der Ansprüche 1 bis 7 umfasst.

13. Bioklebstoffkonstrukt umfassend: einen Träger, der für die Gewebereparatur oder -rekonstruktion geeignet ist; eine erste Beschichtung umfassend ein erstes Multihydroxyphenyl(DHPD)-funktionalisiertes Polymer (DHPp) gemäß einem der Ansprüche 1 bis 7 und ein erstes Polymer; und eine zweite Beschichtung, die auf die erste Beschichtung geschichtet ist, wobei die zweite Beschichtung ein zweites Multihydroxyphenyl(DHPD)-funktionalisiertes Polymer (DHPp) gemäß einem der Ansprüche 1 bis 7 und ein zweites Polymer umfasst, wobei das erste und das zweite Polymer gleich oder verschieden sein können und wobei das erste und das zweite DHPp gleich oder verschieden sein können.

14. Bioklebstoffkonstrukt umfassend: einen Träger, der für die Gewebereparatur oder -rekonstruktion geeignet ist; eine erste Beschichtung umfassend ein erstes Multihydroxyphenyl(DHPD)-funktionalisiertes Polymer (DHPp) gemäß einem der Ansprüche 1 bis 7; und eine zweite Beschichtung, die auf die erste Beschichtung geschichtet ist, wobei die zweite Beschichtung ein zweites Multihydroxyphenyl(DHPD)-funktionalisiertes Polymer (DHPp) gemäß einem der Ansprüche 1 bis 7 umfasst, wobei das erste und das zweite DHPp gleich oder verschieden sein können.

15. Verfahren zum Verringern von Bakterienwachstum auf einer Substratoberfläche, umfassend den Schritt des Beschichtens eines Multihydroxyphenyl(DHPD)-funktionalisierten Polymers (DHPp) gemäß einem der Ansprüche 1 bis 7 auf die Oberfläche des Substrats.

## Revendications

1. Composé de formule (I) 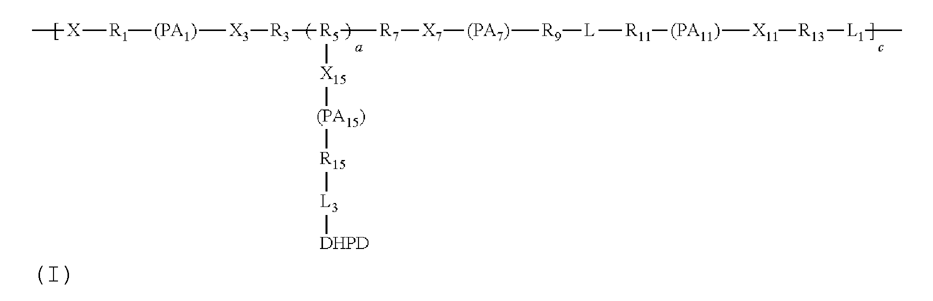 dans laquelle
chaque X, X₃, X₇, X₁₁ et X₁₅ est indépendamment 0 ou NR ; chaque R, s'il est présent, est H ou un groupe alkyle en C₁ à C₁₅ ramifié ou non ramifié ;
chaque R₁, R₃, R₅, R₇, R₉, R₁₁, R₁₃ et R₁₅ est indépendamment un groupe alkyle en C₁ à C₁₅ ramifié ou non ramifié ; chaque PA₁, PA₇, PA₁₁ et PA₁₅ est indépendamment un résidu d'un polyéther sensiblement polyoxyalkyléné ou un dérivé de celui-ci ;
chaque L, L₁ et L₃ est indépendamment un groupe de liaison choisi parmi les liaisons amine, amide, éther, ester, urée, carbonate ou uréthane ;
chaque DHPD est indépendamment l'un parmi la 3,4-dihydroxyphénéthylamine (dopamine), l'acide 3,4-dihydroxyhydrocinnamique, le 3,4-dihydroxyphényléthanol, l'acide 3,4-dihydroxyphénylacétique, la 3,4-dihydroxyphénylamine, et l'acide 3,4-dihydroxybenzoique ;
a vaut de 4 à 8 ; et
c vaut de 3 à 80.

2.Composé selon la revendication 1, dans lequel X est NR et R est H ; et/ou dans lequel chaque X₃, X₇, X₁₁ et X₁₅ est O ; et/ou dans lequel chaque R₁, R₉ et R₁₅ est -CH₂CH₂-.

3. Composé selon la revendication 1 ou 2, dans lequel chaque PA₁, PA₇ et PA₁₅ est indépendamment un résidu d'un polyéther sensiblement polyoxyéthyléné, le nombre de motifs répétitifs du polyéther polyoxyéthyléné étant de préférence de 37, 56 ou 75.

4. Composé selon l'une quelconque des revendications 1 à 3, dans lequel PA₁₁ est un résidu d'un polyéther sensiblement polyoxyéthyléné, le nombre de motifs répétitifs du polyéther polyoxyéthyléné étant de préférence de 10.

5. Composé selon l'une quelconque des revendications 1 à 4, dans lequel chacun de R₃, R₇, R₁₁ et R₁₃ est -CH₂- ; et/ou dans lequel chaque R₅ est -CH ; et/ou dans lequel chaque L, L₁ et L₃ est une liaison amide.

6. Composé selon l'une quelconque des revendications 1 à 5, dans lequel chaque DHPD est un résidu d'acide 3,4-dihydroxyhydrocinnamique (DOHA).

7. Composé selon l'une quelconque des revendications 1 à 6, dans lequel c vaut de 3 à 25.

8. Assemblage bioadhésif comprenant :
un support convenant pour la réparation ou la reconstruction tissulaire ; et
un revêtement comprenant un polymère fonctionnalisé multi-hydroxyphénylique (DHPD) (DHPp) selon l'une quelconque des revendications 1 à 7, comprenant éventuellement en outre un oxydant, l'oxydant étant de préférence formulé avec le revêtement ou appliqué au revêtement.

9. Mélange d'un polymère et d'un composé selon l'une quelconque des revendications 1 à 7, dans lequel le polymère est présent à raison de préférence de 1 à 50 % en poids, mieux encore de 1 à 30 % en poids.

10. Assemblage bioadhésif comprenant : un support convenant pour la réparation ou la reconstruction tissulaire ; et un revêtement comprenant un mélange selon la revendication 9, comprenant éventuellement en outre un oxydant, l'oxydant étant de préférence formulé avec le revêtement ou appliqué au revêtement.

11. Assemblage bioadhésif selon la revendication 8 ou 10, dans lequel le support est un film, un filet, une membrane, un intissé ou un dispositif prosthétique.

12. Assemblage bioadhésif comprenant : un support convenant pour la réparation ou la reconstruction tissulaire ; un premier revêtement comprenant un polymère fonctionnalisé multi-hydroxyphénylique (DHPD) (DHPp) selon l'une quelconque des revendications 1 à 7 et un polymère ; et un deuxième revêtement déposé sur le premier revêtement, le deuxième revêtement comprenant un polymère fonctionnalisé multi-hydroxyphénylique (DHPD) (DHPp) selon l'une quelconque des revendications 1 à 7.

13. Assemblage bioadhésif comprenant : un support convenant pour la réparation ou la reconstruction tissulaire ; un premier revêtement comprenant un premier polymère fonctionnalisé multi-hydroxyphénylique (DHPD) (DHPp) selon l'une quelconque des revendications 1 à 7 et un premier polymère ; et un deuxième revêtement déposé sur le premier revêtement, le deuxième revêtement comprenant un deuxième polymère fonctionnalisé multi-hydroxyphénylique (DHPD) (DHPp) selon l'une quelconque des revendications 1 à 7 et un deuxième polymère, les premier et deuxième polymères pouvant être identiques ou différents, et les premier et deuxième DHPp pouvant être identiques ou différents.

14. Assemblage bioadhésif comprenant : un support convenant pour la réparation ou la reconstruction tissulaire ; un premier revêtement comprenant un premier polymère fonctionnalisé multi-hydroxyphénylique (DHPD) (DHPp) selon l'une quelconque des revendications 1 à 7 ; et un deuxième revêtement déposé sur le premier revêtement, le deuxième revêtement comprenant un deuxième polymère fonctionnalisé multi-hydroxyphénylique (DHPD) (DHPp) selon l'une quelconque des revendications 1 à 7, les premier et deuxième DHPp pouvant être identiques ou différents.

15. Procédé pour réduire la croissance bactérienne sur la surface d'un substrat, comprenant l'étape consistant à déposer un polymère fonctionnalisé multi-hydroxyphénylique (DHPD) (DHPp) selon l'une quelconque des revendications 1 à 7 sous la forme d'un revêtement sur la surface du substrat.
